# EUROPEAN PATENT APPLICATION

(11) **EP 1 541 162 A1**
(43) Date of publication of application: **15.06.2005**
(21) Application number: 03784536.9
(22) Date of filing: 06.08.2003
(51) Int. Cl.: A61K 35/78, A61K 35/74, A61K 9/08, A61P 15/02, A61P 31/04, A61P 31/10, A61P 33/02

(54) **INTRAVAGINAL WASHING AGENT**

(30) Priority: 07.08.2002 JP 2002230034
(71) Applicant: B & S Corporation, Tokyo 102-0076 (JP); Tominaga, Kunihiko, Koriyama-shi, Fukushima 963-8002 (JP)
(72) Inventor: TOMINAGA, Kunihiko, c/o Loma Linda Clinic, Koriyama-shi, Fukushima 963-8002 (JP)
(74) Representative: Ward, David Ian
(86) International application number: PCT/JP2003/009989
(87) International publication number: WO 2004/014408

(57) **Abstract**

An intravaginal washing agent by which the self-cleaning effect in the vagina of a patient suffering from a vaginal infection such as bacterial vaginosis, cervical chlamydiosis, trichomonal vaginitis or candidal vaginitis can be restored in the home, the invasion of bacteria, fungi, protozoa, etc into the vagina can be prevented, and invading organisms can be exterminated. This intravaginal washing agent, which can be easily handled, has no toxicity and exhibits excellent effects, comprises a fermented soybean milk obtained by fermenting soybean milk by coculturing a plural number of lactic acid bacteria or an extract obtained by extracting the fermented soybean milk with an alcohol, in particular, over 6 month or longer.

## Description

### Technical Field

The present invention relates to a washing agent to wash the vagina of a patient suffering from a vaginal infection such as bacterial vaginosis, chlamydial cervicitis, trichomonal vaginitis, or candidal vaginitis, the washing agent also being effective as a curative medicine, and to medical care and pharmaceutical technology.

### Background Art

A large number of fungi of the genus lactic acid bacillus such as Doderlein's bacillus reside in the vagina of healthy adult women who menstruate. The presence of these lactic acid bacilli maintains the healthy balance of bacteria in the vagina and prevents the reproductive organ from being infected by pathogenic microbes.

The lactic acid bacillus is reproduced using glycogen secreted from the vaginal mucosa as an energy source and competes with external pathogens to protect the vaginal cavity from attack by other bacteria. However, because of the recent development of a large number of antibiotics, pharmaceutical preparations of such antibiotics have often been used, causing the lactic acid bacilli in the vagina to be eliminated.

As a result, a large number of infected anaerobic bacteria such as Bacteroides, Peptostreptococcus, Peptococcus, and Mobiluncus G. vaginalis are reproduced in the vagina and interaction between these bacteria causes the development of bacterial vaginitis. Also, the infection of Trichomonas vaginalis or Candida (all of the above Candida albicans) causes the development of vaginitis.

Sulfonamides, antibiotics, and antibacterial agents are chiefly used to cure the above diseases. However, there is a concern that the administration of such antibiotics causes side effects such as rash, pruritus, local reddening, inflammation, and erosion. The administration of such antibiotics also leads to the death of the lactic acid bacilli indwelling in the vagina. Furthermore, since some pathogenic bacteria (for example, Staphylococcus) acquire resistance to the antibiotics, it has been difficult to cure such diseases by administering antibiotics.

Japanese Unexamined Patent Application Publication No. 11-322621 discloses the use of lactic acid bacillus such as Lactobacillus casei, Lactobacillus gasseri, Lactobacillus fermintum, Lactobacillus casei subs. Pseudoplantarum, and Lactobacillus crispatus to cure vaginal infections. This seems to be based on an idea that the lactic acid bacillus is used to replenish the dead bacilli.

Because of the overuse of antibiotics, the decrease in the immune system's function, and the increase in sexual behavior, the number of women suffering from a disease such as bacterial vaginosis, chlamydial cervicitis, trichomonal vaginitis, or candidal vaginitis has been increasing. Many women are reluctant to visit a gynecologist for such a disease and the symptoms are often aggravated.

In order to improve such a situation, the present inventor has considered the known art, developed a washing agent, and performed studies so that the patient can cure the disease by herself, in other words, the patient can wash the vagina by herself. The patient can recover the self-cleaning effect in the vagina using this washing agent in the home. The washing agent prevents bacteria, fungi, and protozoa, etc. from invading into the vagina and sterilizes the invading microorganisms. The washing agent can be easily handled, has no toxicity, and exhibits excellent effects.

As a result, the present inventor has found that substances generally produced and secreted by lactic acid bacteria (hereinafter referred to as an extract of fermentation solution of lactic acid bacteria), which have an effect of improving the condition of intestinal microorganisms, and which are commercially available, solve the above problems. The present inventor has also found that the above substances can be used as an excellent washing agent of the vagina for a woman suffering from bacterial vaginosis, chlamydial cervicitis, trichomonal vaginitis, or candidal vaginitis, and the above substances are effective as a curative medicine for such a vaginal infection. Thus, the present inventor has accomplished the present invention.

### Disclosure of Invention

The invention described in claim 1 of the present invention provides
an intravaginal washing agent composed of fermented soybean milk produced by fermenting soybean milk with a co-culture of a plurality of lactic acid bacteria.

The invention described in claim 2 of the present invention provides
an intravaginal washing agent composed of an extract produced by extracting fermented soybean milk with an alcohol, the fermented soybean milk being produced by fermenting soybean milk with a co-culture of a plurality of lactic acid bacteria.

The invention described in claim 3 of the present invention provides
the intravaginal washing agent composed of an extract produced by the following process. The process includes the steps of sterilizing the fermented soybean milk produced by fermenting soybean milk with a co-culture of a plurality of lactic acid bacteria, adding an alcohol to the fermented soybean milk, and performing the extraction with the alcohol for at least six months.

The invention described in claim 4 of the present invention provides
the intravaginal washing agent composed of an extract produced by extracting fermented soybean milk with an alcohol, the fermented soybean milk being produced by fermenting soybean milk with a co-culture of a plurality of lactic acid bacteria,
wherein the extract includes a total content of at least 20% of an ingredient of which the retention time measured by liquid chromatography under the following conditions is at least 10 minutes.
Measurement conditions for liquid chromatography
Column: Asahipak GS=220H
Mobile phase: 100 mM sodium phosphate buffer
Flow rate: 1.0 /min.
Column temperature: 40°C

The invention described in claim 5 of the present invention provides
the intravaginal washing agent composed of an extract produced by extracting fermented soybean milk with an alcohol, the fermented soybean milk being produced by fermenting soybean milk with a co-culture of a plurality of lactic acid bacteria,
wherein the extract includes a total content of at least 25% of an ingredient of which the retention time measured by liquid chromatography under the following conditions is at least 7 minutes.
Measurement conditions for liquid chromatography
Column: Asahipak GS=220H
Mobile phase: 100 mM sodium phosphate buffer
Flow rate: 1.0 /min.
Column temperature: 40°C

The invention described in claim 6 of the present invention provides
the intravaginal washing agent composed of an extract produced by extracting fermented soybean milk with an alcohol, the fermented soybean milk being produced by fermenting soybean milk with a co-culture of a plurality of lactic acid bacteria,
wherein the extract includes a total content of at least 25% of an ingredient of which the retention time measured by liquid chromatography under the following conditions is at least 7 minutes, and a total content of at least 20% of an ingredient of which the retention time measured by liquid chromatography under the following conditions is at least 10 minutes.
Measurement conditions for liquid chromatography
Column: Asahipak GS=220H
Mobile phase: 100 mM sodium phosphate buffer
Flow rate: 1.0 /min.
Column temperature: 40°C

### Brief Description of the Drawings

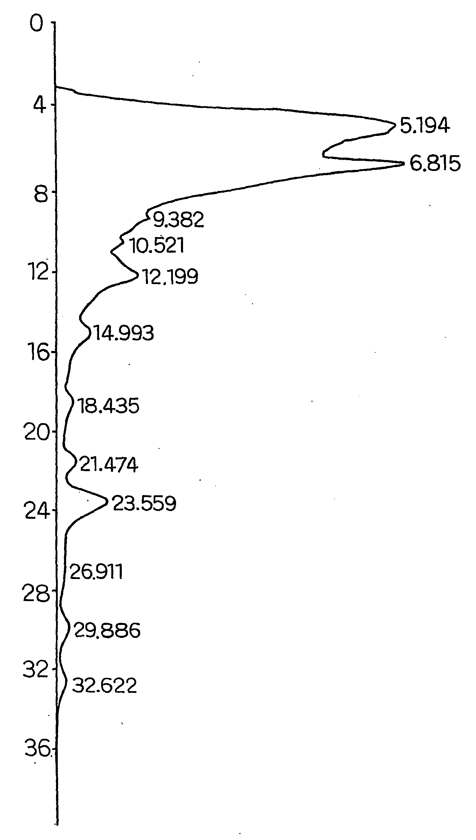

Fig. 1 is a chart from a liquid chromatograph of an extract of fermentation solution of lactic acid bacteria according to the present invention.

### Best Mode for Carrying Out the Invention

An intravaginal washing agent according to the present invention includes fermented soybean milk as the active ingredient. The fermented soybean milk is produced by fermenting soybean milk with a co-culture of a plurality of lactic acid bacteria, preferably, for about 100 to about 300 hours. This fermented soybean milk itself can be used as the intravaginal washing agent in the present invention.

More specifically, examples of the active ingredient of the intravaginal washing agent include a substance containing an extract of fermented soybean milk and other various impurities, and a solid or a liquid prepared by solid-liquid separation and containing the extract of fermented soybean milk. In order to utilize the extract of fermented soybean milk effectively, the extract used as the effective ingredient is preferably extracted with an organic solvent. More preferably, the extract is produced by extracting fermented soybean milk with an alcohol, the fermented soybean milk being produced with a co-culture of a plurality of lactic acid bacteria.

It is known that substances generally produced and secreted by lactic acid bacteria, which are contained in fermented soybean milk produced by fermentation with a co-culture (hereinafter referred to as fermented soybean milk extract), have a superior advantage for human health. In addition, many products containing such substances are commercially available.

The fermented soybean milk extract in the present invention is obtained from fermented soybean milk produced by fermenting soybean milk with a co-culture of a plurality of lactic acid bacteria, and in particular, is obtained by extracting the fermented soybean milk with an alcohol. The conditions for the extraction with an alcohol are as follows:
(1) The alcohol is preferably ethanol because the fermented soybean milk extract is used as a food. An approximately equivalent amount of ethanol is added to the fermented soybean milk, and the fermented soybean milk is extracted.
(2) Sterilized fermented soybean milk is used for the extraction. Examples of the sterilization method include heat sterilization and sterilization by disrupting the bacterial cells.

Although the extraction may be performed with heating, the extraction is preferably performed at normal temperature to prevent thermal denaturation. In addition, the extraction is preferably performed in a cool, dark place to prevent the long-term influence of light.

It is expected that the extraction at normal temperature proceeds at a low speed. In order to produce a superior extract from the fermented soybean milk, the extraction is performed for a long time, generally for at least 6 months, and preferably at least 1 year. Taking such a long time provides a superior fermented soybean milk extract.

Fermented soybean milk produced by fermentation with a co-culture of a plurality of lactic acid bacteria is sterilized, and an alcohol is then added to the fermented soybean milk. The extraction is performed for at least 6 months. As will be clarified in the following Examples, this process provides the following fermented soybean milk extract and also increases the kind of the ingredient. The fermented soybean milk extract includes a total content of at least 20%, preferably at least 25%, of an ingredient of which the retention time measured by liquid chromatography under a specific condition is at least 10 minutes.

### The ingredient is defined in more detail as follows:

The fermented soybean milk extract includes a total content of at least 25%, preferably at least 30%, of an ingredient of which the retention time measured by liquid chromatography is at least 7 minutes; or the fermented soybean milk extract includes a total content of at least 25%, preferably at least 30%, of an ingredient of which the retention time measured by liquid chromatography is at least 7 minutes and a total content of at least 20%, preferably at least 25%, of an ingredient of which the retention time measured by liquid chromatography is at least 10 minutes.
As the extraction is performed for a long time, the amount of each ingredient in the fermented soybean milk extract gradually increases. However, since the long-term extraction decreases the extraction effect and decreases the efficiency of the extraction operation, a fermented soybean milk extract generally containing each ingredient up to 40% and up to 35%, preferably containing up to 35% and up to 30% is used.

In order to clean the vaginal walls, the fermented soybean milk or the extract thereof that is the intravaginal washing agent of the present invention can be used as follows without further treatment or it can be diluted. The fermented soybean milk or the extract thereof may be applied on the pudendum. For example, gauze soaked with the fermented soybean milk or the extract thereof may be applied on the affected area. Furthermore, the fermented soybean milk or the extract thereof may be introduced and dispersed in the vagina with a dropping pipette, and a cotton ball or a tampon may then be inserted for some time.

The fermented soybean milk or the extract thereof in the present invention does not impose a strict limitation on the quantity applied, and can be freely used, because it is widely ingested as a health maintenance food and does not cause side effects. Therefore, the intravaginal washing agent of the present invention may be used everyday, every other day, or every few days. As a result, the use of the intravaginal washing agent cleans the inside of the vagina to alleviate or terminate the symptoms of, for example, bacterial vaginosis, chlamydial cervicitis, trichomonal vaginitis, or candidal vaginitis.

### <EXAMPLES>

The intravaginal washing agent of the present invention will now be described in more detail with reference to the Examples.

### EXAMPLE 1

### <Preparation extract of fermentation solution>

As shown in the following Table 1, lactic acid bacteria and yeast that form combinations of four kinds and four groups were prepared. In the table, symbols A, B, 1, 2, 3, and 4 represent that the strain is different.

**Table 1**

| | (1) | (2) | (3) | (4) |
|---|---|---|---|---|
| I | B. bulgaricus A | B. acidophilus 1. | Micrococcus lactisacidi 1. | Yeast 1. |
| II | B. bulgaricus B | B. acidophilus 2. | Micrococcus lactisacidi 2. | Yeast 2. |
| III | Kornchenbacillus A | B. acidophilus 3. | Micrococcus lactisacidi 3. | Yeast 3. |
| IV | Kornchenbacillus B | B. acidophilus 4. | Micrococcus lactisacidi 4. | Yeast 4. |

The lactic acid bacteria in each group were separately cultured for 48 hours using soybean milk as the culture medium, while the temperature was gradually increased from 20°C to 40°C.

After the culture was performed for 48 hours, the culture solutions were transferred to one container. The mixture was further cultured for 96 hours, while the temperature was gradually increased from 20°C to 40°C.

The fermented soybean milk prepared by completing the culture was sterilized by heating. Subsequently, an equivalent amount of alcohol was added to the solution. The mixture was kept in the cool and dark place for one year to perform the extraction.

The extracted fermented soybean milk was filtered and subjected to solid-liquid separation to prepare a clear light yellow fermented soybean milk extract I.

The resultant extract of fermentation solution was analyzed by liquid chromatography under the following conditions. Table 2 and Fig. 1 show the results.
Measurement conditions for liquid chromatography
Column: Asahipak GS=220H
Mobile phase: 100 mM sodium phosphate buffer
Flow rate: 1.0 /min.
Column temperature: 40°C
Detector: Ultraviolet spectrophotometer
Detection wavelength: 210 nm

**Table 2**

| ingredient | retention time | area | height | concentration |
|---|---|---|---|---|
| 1 | 5.194 | 8974013 | 71553 | 37.8998 |
| 2 | 6.815 | 7031508 | 73112 | 29.6960 |
| 3 | 9.382 | 1118511 | 19495 | 4.7238 |
| 4 | 10.521 | 559947 | 14058 | 2.3648 |
| 5 | 12.199 | 2181670 | 17105 | 9.2138 |
| 6 | 14.993 | 908867 | 7338 | 3.8384 |
| 7 | 18.435 | 445615 | 3782 | 1.8820 |
| 8 | 21.474 | 362632 | 4422 | 1.5315 |
| 9 | 23.559 | 1158857 | 10852 | 4.8942 |
| 10 | 26.911 | 237078 | 2011 | 1.0012 |
| 11 | 29.886 | 358612 | 2975 | 1.5145 |
| 12 | 32.622 | 340970 | 2516 | 1.4400 |
| total | | 23678272 | 229218 | 100.0000 |

### <Confirmatory test of cleaning effect-1>

After written informed consent was obtained from seven female outpatients (19 years old to 29 years old) who visited a gynecologist complaining of an increase in vaginal discharge and an unpleasant odor therefrom, 1 cc of the above extract of fermentation solution was evenly dispersed on the vaginal walls with a dropping pipette under the direct observation of the inside of the vagina. Subsequently, a cotton ball sterilized by high pressure was inserted in the vagina. Four hours later, the cotton ball was removed by the patients.

When the intravaginal cleaning was performed, vaginal discharge in the vagina was collected to check for Chlamydia, Trichomonas, and Candida. Patients who were negative for Chlamydia, Trichomonas, and Candida, and had vaginal discharge that was yellow and had an amine-like odor were clinically diagnosed as having bacterial vaginosis. Among the seven patients, five women were diagnosed with bacterial vaginosis, one woman was diagnosed with chlamydial cervicitis, and one woman was diagnosed with candidal vaginitis.

The cleaning effects in the patients were as follows. The duration of the test was March to April in 2002.
(1) Patient (A.M.) 20 years old, Bacterial vaginosis
   March 28, First cleaning, A large amount of yellow vaginal discharge, Strong odor
   March 30, Second cleaning, Both the vaginal discharge and the odor were somewhat decreased. (Two days after the first cleaning)
   April 6, Third cleaning, Both the vaginal discharge and the odor were improved by 100%. (Eight days after the first cleaning)
   April 9, Fourth cleaning, The condition in which both the vaginal discharge and the odor were improved by 100% was maintained.
(2) Patient (F.Y.) 29 years old, Bacterial vaginosis
   April 9, First cleaning, A large amount of yellow vaginal discharge, Strong odor
   April 11, Second cleaning, Remained the same, (Two days after the first cleaning)
   April 13, Third cleaning, Both the vaginal discharge and the odor were improved by 50%. (Four days after the first cleaning)
   April 15, Fourth cleaning, The condition in which both the vaginal discharge and the odor were improved by 50% was maintained.
(3) Patient (Y.E.) 19 years old, Bacterial vaginosis
   April 9, First cleaning, A large amount of yellow vaginal discharge, Strong odor
   April 11, Second cleaning, The vaginal discharge and the odor remained the same, (Two days after the first cleaning)
   April 15, Third cleaning, The vaginal discharge was decreased by 30%, and the odor was improved by 40%.
(4) Patient (Y.C.) 24 years old, Bacterial vaginosis
   March 8, First cleaning, A large amount of yellow vaginal discharge, Strong odor
   March 12, Second cleaning, Both the vaginal discharge and the odor were somewhat decreased. (Four days after the first cleaning)
   March 15, Third cleaning, The vaginal discharge was improved by 50%, and the odor was improved by 100%.
   March 22, Fourth cleaning, The condition in which the vaginal discharge was improved by 50% and the odor was improved by 100% was maintained.
(5) Patient (K.H.) 29 years old, Bacterial vaginosis
   March 5, First cleaning, A large amount of yellow vaginal discharge, Strong odor
   March 11, Second cleaning, Both the vaginal discharge and the odor were somewhat decreased. (Six days after the first cleaning)
   March 14, Third cleaning, The vaginal discharge and the odor were further improved. (Nine days after the first cleaning)
   March 22, Fourth cleaning, Both the vaginal discharge and the odor were improved by 100%.
(6) Patient (M.S.) 21 years old, Chlamydial cervicitis
   April 6, First cleaning, A large amount of yellow vaginal discharge, Strong odor
   April 8, Second cleaning, Both the vaginal discharge and the odor were somewhat decreased. (Two days after the first cleaning)
   April 9, Third cleaning, The vaginal discharge was improved by 40%, and the odor was improved by 100%.
   April 12, Fourth cleaning, The condition in which the vaginal discharge was improved by 40% and the odor was improved by 100% was maintained.
(7) Patient (S.M.) 27 years old, Candidal vaginitis
   March 8, First cleaning, A large amount of white vaginal discharge
   March 11, Second cleaning, The vaginal discharge was somewhat decreased. (Three days after the first cleaning)
   March 14, Third cleaning, The vaginal discharge was decreased by 80%. (Six days after the first cleaning)
   April 15, Fourth cleaning, The condition in which both the vaginal discharge and the odor were improved by 50% was maintained.

### <Confirmatory test of cleaning effect-2>

The following symptoms were tested by outpatients who mainly complained of vaginal discharge with an unpleasant odor.
1. Gray vaginal discharge
2. pH in the vagina>4.5 (5.0)
3. Detection of an amine-like odor
4. Detection of a clue cell (at least 20% of the epithelial cells)
Among 30 patients who were positive for the three items out of the above symptoms, 23 patients who could visit the hospital for treatment for one week were selected. After written informed consent was obtained from the patients, the above-mentioned extract of fermentation solution was administered in the vagina. On the other hand, Chlomy (registered trademark) vaginal tablets (chloramphenicol vaginal tablets) were administered to seven patients who could not visit the hospital.

The degree of the unpleasant odor and the vaginal discharge was evaluated by the test subjects as outpatients using a visual analog scale (VAS).

Table 3 shows the evaluation results of the patients to whom the extract of fermentation solution was administered. Table 4 shows the evaluation results of the patients to whom Chlomy vaginal tablets were administered. The evaluation showed the following results.
(1) In the patient group to whom the extract of fermentation solution was administered in the vagina, one week after the administration, the degree of the unpleasant odor and the vaginal discharge was significantly decreased (P<0.001 and P<0.001, respectively), compared with that before the administration.
(2) In the patient group to whom Chlomy vaginal tablets were administered, one week after the administration, the degree of the unpleasant odor and the vaginal discharge was significantly decreased (P=0.0016 and P=0.0018, respectively), compared with that before the administration.
(3) The change in the degree of the unpleasant odor and the vaginal discharge (decreasing ratio %) was represented by formula [(before administration - after administration) / before administration]. In the above decreasing ratio, there was no statistically significant difference between the group to whom the extract of fermentation solution was administered in the vagina and the group to whom Chlomy vaginal tablets were administered.
(4) In conclusion, the administration of the fermentation solution extract in the vagina had the same curative effect as that of Chlomy vaginal tablets and caused no side effects.

**Table 3**

| patient | age | first examination | | one week later | |
|---|---|---|---|---|---|
| | | vaginal discharge | unpleasant odor | discharge /decreasing rate(%) | odor /decreasing rate(%) |
| 1 | 20 | 100 | 95 | 22/78 | 18/81 |
| 2 | 35 | 75 | 79 | 23/69 | 38/52 |
| 3 | 25 | 60 | 84 | 21/65 | 0/100 |
| 4 | 25 | 64 | 64 | 18/72 | 6/91 |
| 5 | 35 | 76 | 48 | 31/59 | 19/60 |
| 6 | 24 | 33 | 70 | 4/88 | 6/91 |
| 7 | 22 | 75 | 79 | 23/69 | 38/52 |
| 8 | 20 | 100 | 30 | 22/78 | 26/13 |
| 9 | 20 | 100 | 95 | 22/78 | 18/81 |
| 10 | 21 | 43 | 42 | 3/93 | 0/100 |
| 11 | 18 | 54 | 67 | 0/100 | 0/100 |
| 12 | 28 | 70 | 91 | 46/34 | 61/33 |
| 13 | 20 | 54 | 67 | 0/100 | 0/100 |
| 14 | 21 | 52 | 65 | 0/100 | 0/100 |
| 15 | 25 | 72 | 21 | 21/71 | 5/76 |
| 16 | 25 | 62 | 65 | 0/100 | 0/100 |
| 17 | 25 | 60 | 84 | 0/100 | 0/100 |
| 18 | 38 | 60 | 63 | 0/100 | 0/100 |
| 19 | 27 | 61 | 64 | 12/80 | 0/100 |
| 20 | 20 | 72 | 76 | 0/100 | 0/100 |
| 21 | 21 | 54 | 67 | 22/59 | 0/100 |
| 22 | 25 | 76 | 48 | 0/100 | 0/100 |
| 23 | 37 | 54 | 49 | 4/93 | 4/92 |

**Table 4**

| patient | Age | first examination | | one week later | |
|---|---|---|---|---|---|
| | | vaginal discharge | Unpleasant odor | discharge /decreasing rate(%) | odor /decreasing rate(%) |
| 1 | 23 | 73 | 65 | 12/84 | 10/84 |
| 2 | 28 | 91 | 70 | 21/77 | 42/40 |
| 3 | 22 | 79 | 75 | 14/82 | 11/85 |
| 4 | 18 | 47 | 55 | 17/64 | 34/38 |
| 5 | 37 | 100 | 71 | 0/100 | 23/68 |
| 6 | 33 | 64 | 75 | 15/77 | 45/40 |
| 7 | 21 | 58 | 50 | 43/26 | 34/32 |
| 8 | 21 | 91 | 92 | 1/99 | 0/100 |

### Industrial Applicability

Ingredients in fermented soybean milk produced by fermenting soybean milk with a co-culture of a plurality of lactic acid bacteria, particularly in the above fermented soybean milk extract have been identified to a certain degree. Although nucleic acids, amino acids, and vitamins are listed as the ingredients, some ingredients are still unknown.

The following function of the extract of fermented soybean milk is known: The extract of fermented soybean milk improves the condition of intestinal microorganisms and helps, for example, the synthesis of hormones, the synthesis of vitamins and enzymes, metabolism of cholesterol, blood sugar regulation, and blood pressure homeostasis. In addition, the extract of fermented soybean milk is effective against hepatitis, chronic arthritis, atopic dermatitis, angina pectoris, and various cancers. However, only a few pharmacologic effects are specifically confirmed under the present situation. At present, the mechanism of action of the effects in the present invention is also not known.

However, as shown in the above clinical results, the fermented soybean milk or the extract thereof that is the intravaginal washing agent of the present invention is an effective medical agent (washing agent) against bacterial vaginosis, chlamydial cervicitis, and candidal vaginitis, and in addition, the effect works immediately. About three days after the administration, the symptoms were clearly improved and this condition was maintained. Even when the interval of the administration was one week or more, the effect could be maintained. Thus, the fermented soybean milk or the extract thereof is a superior medical agent.

These advantages are significantly excellent, compared with a known tablet of lactic acid bacteria having an effective ratio of about 60% and a known yogurt containing lactic acid bacteria having an effective ratio of about 50%, the yogurt causing incongruity or discomfort when used as a washing agent. When the curative effect was finally assessed 30 days later, the fermented soybean milk or the extract thereof had excellent effects, compared with, for example, lactic acid produced by lactic acid bacteria. In this case, there was also a significant difference in the improvement in the surface of the skin.

In addition, the fermented soybean milk or the extract thereof that is the intravaginal washing agent in the present invention had the same curative effect as that of commercially available Chlomy vaginal tablets and had no side effects.

As clearly described above, the substances generally produced and secreted by lactic acid bacteria (hereinafter referred to as an extract of fermentation solution of lactic acid bacteria), which have an effect of improving the condition of intestinal microorganisms, and which are commercially available, can be used as an excellent washing agent of the vagina of a woman suffering from bacterial vaginosis, chlamydial cervicitis, trichomonal vaginitis, or candidal vaginitis. Furthermore, the above substances are effective as a curative medicine for such a vaginal infection. These substances can be widely used in the field of medical care, such as in medicines and pharmaceuticals.

## Claims

1. An intravaginal washing agent comprising:
fermented soybean milk produced by fermenting soybean milk with a co-culture of a plurality of lactic acid bacteria.

2. An intravaginal washing agent comprising:
an extract produced by extracting fermented soybean milk with an alcohol, the fermented soybean milk being produced by fermenting soybean milk with a co-culture of a plurality of lactic acid bacteria.

3. The intravaginal washing agent according to claim 2,
wherein the extraction with an alcohol is performed by adding an alcohol to the sterilized fermented soybean milk and is performed for at least six months.

4. The intravaginal washing agent according to claim 2 or claim 3,
wherein the extract includes a total content of at least 20% of an ingredient of which the retention time measured by liquid chromatography under the following conditions is at least 10 minutes.
Measurement conditions for liquid chromatography
Column: Asahipak GS=220H
Mobile phase: 100 mM sodium phosphate buffer
Flow rate: 1.0 /min.
Column temperature: 40°C

5. The intravaginal washing agent according to claim 2 or claim 3,
wherein the extract includes a total content of at least 25% of an ingredient of which the retention time measured by liquid chromatography under the following conditions is at least 7 minutes.
Measurement conditions for liquid chromatography
Column: Asahipak GS=220H
Mobile phase: 100 mM sodium phosphate buffer
Flow rate: 1.0 /min.
Column temperature: 40°C

6. The intravaginal washing agent according to claim 2 or claim 3,
wherein the extract includes a total content of at least 25% of an ingredient of which the retention time measured by liquid chromatography under the following conditions is at least 7 minutes, and a total content of at least 20% of an ingredient of which the retention time measured by liquid chromatography under the following conditions is at least 10 minutes.
Measurement conditions for liquid chromatography
Column: Asahipak GS=220H
Mobile phase: 100 mM sodium phosphate buffer
Flow rate: 1.0 /min.
Column temperature: 40°C
